# EUROPEAN PATENT APPLICATION

(11) **EP 1 941 926 A2**
(43) Date of publication of application: **09.07.2008**
(21) Application number: 07119360.1
(22) Date of filing: 26.10.2007
(51) Int. Cl.: A61M 15/00, A61M 15/02

(54) **Apparatus and Method for producing salt, preferably NaCl aerosol suitable for treating respiratory diseases**

(30) Priority: 08.01.2007 HU 0700007
(71) Applicant: Asthma Rehabilitációs Centrum KFT., 4200 Hajdúszoboszló (HU)
(72) Inventor: BOZÓKY, Dénes, H-4200, Hajdúszoboszló (HU); TOROKTIN, Alexander, Uzsgorod (UA)
(74) Representative: Kovacs, Ivanné

(57) **Abstract**

Object of the present invention is an apparatus for producing salt aerosol, preferably NaCl aerosol suitable for treating respiratory diseases, the apparatus comprising a ventilator, a delivery pipeline connected to the output of the ventilator, and the pipeline is connected to a throat for entering ground salt containing NaCl salt into the air streaming in the delivery pipeline for producing aerosol as a mixture of salt and air.

The essence of the present invention lays in that in the pipeline section being between the output of ventilator (1) and the pipeline connection to throat (5) the delivery pipeline (2) is provided with a heating device (4) and at the output of delivery pipeline (2) a collisional-ionization arrangement (7) is placed.

A further object of the present invention is a method for operating the apparatus according to the invention.

The essence of the method is switching on the heating device for a given time for heating the aerosol. Than switching on the ventilator and the grinding mill, setting the flow rate of the aerosol comprising air and ground salt particles and the aerosol streaming towards the steel grids assembly of the collisional-ionization arrangement to about 27 m/sec so that the temperature being 100 - 130°C at the beginning of the air blast and decreasing to 36 - 40°C during cca. 1,5 minutes to the end of the air blast. Switching off the ventilator, the grinding mill and the heating device after some minutes', preferably after one and a half to three and a half minutes' operation, then allowing the aerosol to be spread in the air for 15 minutes before beginning of the treatment and carrying out the treatment preferably for 15 - 60 minutes.

## Description

The present invention relates to a method and apparatus for producing ionized NaCl aerosol with high dispersity being suitable for treating anhelous patients, preferably for treating respiratory allergy, trachitis, asthma, etc.

In treating different respiratory diseases there are a lot of various additional treatments using sodium chloride (NaCl). During these treatments saltpipes are applied, artificial salt caves are established or vaporized salt solutions are used.

A common feature of the vaporized salt solutions lies in having their effect on the upper respiratory tracts only without reaching the bronchia in the lower tract of the lungs because the salt particles of the vaporized salt solution adhere to the upper respiratory tracts owing to the great humidity.

The salt never runs out as the patient inhales mostly air.

There is not any active agent in the artificial salt caves. In the natural salt caves there are some but only in case the patients' walking swirl them from the ground.

The aim of the present invention is to develop an apparatus and a method for treating respiratory diseases by means of an active agent, in this case using ionized NaCl aerosol with high dispersity, penetrating into the bronchia being in a spasmodic and inflamed condition.

The recognition of the present invention is to produce ionized NaCl aerosol with high dispersity by grinding the salt to a given particle size, mixing the salt particles to hot air flow, than streaming the mixture towards a metal grid assembly preferably made of steel for a collisional-ionization on the grid assembly, and than forwarding the mixed product into the curing chamber.

Object of the present invention is thus an apparatus for producing NaCl aerosol preferably for treating respiratory diseases. The apparatus comprising a ventilator, a delivery pipeline system connected to the output of the ventilator, in the pipeline system being a pipeline section having a pipeline connection for entering the ground salt containing NaCl into the air stream in the delivery pipeline and for producing aerosol as a mixture of salt and air.

The essence of the apparatus lies in that in the pipeline section between the output of the ventilator and the pipeline connection to the throat a heating device is arranged and at the output of the delivery pipeline a collisional-ionization arrangement is placed. The output of the collisional-ionization arrangement is the output of the apparatus opening into the curing chamber.

According to a preferred embodiment of the present invention the heating device comprising preferably four heating elements being placed in the delivery pipeline angular to the longitudinal axis thereof or the heating device comprising heating elements placed inside concentric along the periphery of the delivery pipeline.

According to another preferred embodiment of the present invention the heating device is developed for heating the aerosol to 100 - 150 °C in the delivery pipeline.

According to a further preferred embodiment of the present invention the grinding mill is developed for grinding most of the salt particles to a size of 0,5-5 micron.

According to another preferred embodiment of the present invention the collisional-ionization arrangement is a grid assembly comprising grids arranged in sequence, further on the grids of the collisional-ionization arrangement having openings in quadratic structure, and the grid elements being shifted relating to that of the previous grid and the grids preferably being steel grids.

Another object of the present invention relates to a method for operating the apparatus according to the invention. The essence of the method is switching on the heating device for a predetermined time for heating the aerosol than switching on the ventilator and the grinding mill, controlling the flow rate of the aerosol streaming through the collisional-ionization arrangement and comprising air and ground salt particles to about 27m/sec, controlling the temperature of the aerosol in a way that being 100 - 150°C at the beginning and 36 - 40°C at the end of the air blast; and after some minutes', preferably after 3 and a half minutes' operation switching off the ventilator, the grinding mill and the heating device than allowing to widespread the emerged aerosol in the curing chamber for 15 minutes before beginning the treatment and carrying out the treatment preferably for 15 - 60 minutes.

The invention will now become better apparent from the following detailed description of an embodiment of the invention and illustrated in the accompanying drawing, wherein:

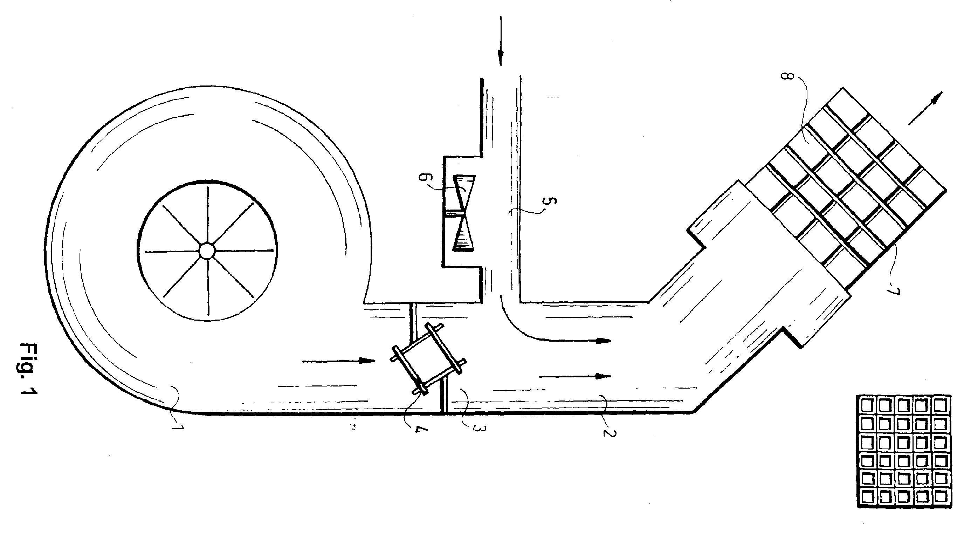

Fig. 1 shows a schematic drawing of a preferred embodiment according to the invention.

In Fig. 1 a ventilator 1 is shown with an output connected to a delivery pipeline 2 optionally formed with a square-like cross section the dimension of which is 125 x 125 mm in the present case. It is practically a chimney within which the salt particles mixed with air will be delivered to the patient. The power of the ventilator is 0,37 kW, the air delivery rate is 1000 m³/hour and the rotational speed of the ventilator is 2800 RPM (rotation per minute).

In the first section of delivery pipeline 2 a heating device 3 is arranged near to the output of ventilator 1, the heating device 2 comprising several resistance heating cable, preferably four heating elements 4, if necessary. The power of heating device 3 i.e. that of the four heating elements 4 is preferably 2400 - 3200 Watt. The heating device 3 is placed either inwardly in delivery pipeline 2 angular with its longitudinal axis or arranged as a heating jacket on the side-walls of delivery pipeline 2. Other heating assemblies known per se can, of course, also be used. In the second section of delivery pipeline 2 following heating device 3there is a pipeline coupling to the output of a throat 5, said output having rectangular cross-section, too, and being preferably dimensioned: 85 X 30 mm. The input of throat 5 is the outer atmosphere. Perpendicular to the axis of the section between the input and output of throat 5 a grinding mill 6 is connected into said throat 5. The cross-section of pipeline 2 and that of the throat can be circular, too. The salt is ground in the grinding mill 6. The rotational speed of the grinding knife of grinding mill 6 is 10000 - 20000 RPM (rotation per minute), preferably 10000 - 12000 RPM, the diameter of its axis is 8 - 10 cm, and its height is 6 - 8 cm. In grinding mill 6 most of the salt is ground to particle size of 0,5 - 5 microns.

In delivery pipeline 2 following throat 5 the aerosol streaming towards the output of delivery pipeline 2 is the mixture of hot salt particles and air.

On the output of delivery pipeline 2 there is a collisional-ionization arrangement 7 comprising a sequence of steel grids 8 having openings of quadratic cross-section, and in each steel grid 8 the grid elements are shifted relating to that of the previous grid 8. Size of the openings in the grids is 15 X 15 mm, wall thickness of the steel sheets is 1,5 mm, height of a layer constituted by the grids, is 15 mm; and - as shown sidewards - four layers of steel grids 8 are placed sequential having an interspace of 20 mm between the layers, and - as mentioned above - the grid elements of grids 8 in sequence are shifted in plane relative to each other in order to make the collision more effective. Measure of shifting is 5 mm. Instead of steel grids 8 other metal grids can also be applied, such as titanium.

The high temperature aerosol and the shifted grid elements of steel grids 8 in sequence being in collisional-ionization arrangement 7 collectively ensure more effective collision and ionization of the salt particles. Inhaling the air comprising said ionized salt particles of 0,5 - 5 microns being the climate of the treating chamber, the salt particles enter into the deepest parts of the lungs, into the bronchia, too, thus the treatment is more effective than any other therapy known until now.

Operation of the apparatus according to the present invention is, as follows.

The first step is switching on heating device 3 in order to bring up to red heat heating elements 4 (cca. 90 - 120 sec). The rated power of heating elements 4 is preferably cca. 2,4 kW.

Next step is switching on ventilator 1 and grinding mill 6.

The flow rate of the aerosol comprising air and ground salt particles and streaming towards steel grids assembly 8 of collisional-ionization arrangement 7, is about 27 m/sec. The temperature of the aerosol is at the beginning of the air blast 100 - 130°C and decreasing to 36 - 40°C to the end of the air blast. After getting into the airspace the aerosol takes on the temperature of the air being in the chamber. The device is operating 1,5 - 3,5 minutes. Then switching off ventilator 1, grinding mill 6 and heating device 3, too.

Allowing the aerosol to be spread in the air for 15 minutes in order that the large-sized salt particles with a diameter of over 5-8 microns can be deposited, and only the particles with a diameter of 0,5 - 5 microns remain afloat. Allowing the aerosol to stand is needed because the diameter of the inlet opening of the bronchia is cca. 5 microns thus the particles being greater than 5 microns can either not get in. At least 93 % of the salt particles have a diameter of 0,5 - 5 microns at this stage. This climate can be preserved for about 1,5 - 2 hours. The treatment can be carried out during this period. The quantity of the active agent being present in this climate is 7 - 9 mg/m³.

It is advisable to stay in this climate for 15 - 60 minutes, and all together 20 treatments are recommended.

Our apparatus differs essentially from any others indeed as neither of the known apparatus produces ionized NaCl aerosol with high dispersity.

For the composition of the aerosol following example is given. Other salts can, of course, be applied essential is the appropriate NaCl amount.

| | |
|---|---|
| Na⁺ | 38-39- % |
| Ca²ⁿ⁺ | 0,2-0,8 %, preferably 0,4-0,7 % |
| Mg²⁺⁺ | max. 0,15 %, preferably cca. 0,1 % |
| Cl⁻ | cca. 60 %, preferably 59-60 % |
| SO₄⁻ | 1-1,5 %, preferably 1-1,2 %. |

The rest is insoluble agent.

## Claims

1. Apparatus for producing salt aerosol, preferably NaCl aerosol suitable for treating respiratory diseases, the apparatus comprising a ventilator, a delivery pipeline connected to the output of the ventilator, the pipeline is connected to a throat for entering ground salt containing NaCl salt into the air streaming in the delivery pipeline for producing aerosol as a mixture of salt and air **characterized by that** in the pipeline section being between the output of ventilator (1) and the pipeline connection to throat (5) the delivery pipeline (2) is provided with a heating device (4) and at the output of delivery pipeline (2) a collisional-ionization arrangement (7) is placed.

2. Apparatus according to claim 1 **characterized by** that the heating device (3) being arranged in delivery pipeline (2) angular to the longitudinal axis thereof comprising preferably four heating elements (4).

3. Apparatus according to claim 1 **characterized by that** heating device (3) comprising heating elements (4) arranged inside of delivery pipeline (2) concentric along the periphery thereof.

4. Apparatus according to claim 1 **characterized by that** heating device (3) is developed for heating the aerosol to 100 - 150 °C in delivery pipeline (2).

5. Apparatus according to claim 1 **characterized by that** grinding mill (6) is developed for grinding most part of the salt particles to a size of 0,5-5 micron.

6. Apparatus according to claim 1 **characterized by that** the collisional-ionization arrangement (7) comprising a grid assembly consisting of sequence of grids (8).

7. Apparatus according to claim 6 **characterized by** that the grids of the collisional-ionization arrangement (7) having quadratic openings in grid structure, and the grid elements of a grid (8) are shifted relating to that of the previous grid (8).

8. Apparatus according to claim 7 **characterized by that** the collisional-ionization arrangement (7) comprising steel grids (8).

9. Method for operating the apparatus according to any of the preceding claims **characterized by that** switching on the heating device for a given time for heating the aerosol than switching on the ventilator and the grinding mill, setting the flow rate of the aerosol comprising air and ground salt particles and the aerosol streaming towards the steel grids assembly of collisional-ionization arrangement to about 27 m/sec so that the temperature being 100 - 130°C at the beginning of the air blast and decreasing to 36 - 40°C during cca. 1,5 minutes to the end of the air blast; switching off the ventilator, the grinding mill and the heating device after some minutes', preferably after one and a half to three and a half minutes' operation, then allowing the aerosol to spread in the air for 15 minutes before beginning of the treatment and carrying out the treatment preferably for 15 - 60 minutes.
